# EUROPEAN PATENT SPECIFICATION

(11) **EP 1 073 683 B1**
(45) Date of publication and mention of the grant of the patent: **28.12.2005**
(21) Application number: 99918932.7
(22) Date of filing: 30.04.1999
(51) Int. Cl.: C07K 16/28, C12N 15/08, C12N 15/13, C12N 15/63, C07H 21/04, A61K 39/395

(54) **G-CSF RECEPTOR AGONIST ANTIBODIES AND SCREENING METHOD THEREFOR**
G-CSF-REZEPTORAGONIST-ANTIKÖRPER UND METHODE ZU IHRER ISOLIERUNG MITTELS DURCHMUSTERUNG
ANTICORPS AGONISTES POUR LE RECEPTEUR G-FSC ET PROCEDE DE CRIBLAGE ASSOCIE

(30) Priority: 30.04.1998 US 83575 P
(43) Date of publication of application: 07.02.2001
(73) Proprietor: Tanox, Inc., Houston, TX 77025 (US)
(72) Inventor: NI, Baofu, Houston, TX 77096 (US); SUN, Bill, N., C., Bellaire, TX 77041 (US); SUN, Cecily, R., Y., Bellaire, TX 77041 (US)
(74) Representative: Vossius & Partner
(86) International application number: PCT/US1999/009466
(87) International publication number: WO 1999/055735

(56) References cited:
- WO-A-95/21864
- WO-A-95/29690
- WO-A-97/12977
- US-A- 5 362 853
- US-A- 5 506 107
- US-A- 5 581 476
- US-A- 5 665 863
- LAYTON J.E. et al., "Identification of a Functional Domain of Human Granulocyte Colony-Stimulating Factor Using Neutralizing Monoclonal Antibodies", J. BIOLOGICAL CHEMISTRY, 15 December 1991, Vol. 266, No. 35, pages 23815-23823, XP002919518.
- LAYTON J.E. et al., "Neutralising Antibodies to the Granulocyte Colony-Stimulating Factor Receptor Recognise Both the Immunoglobulin-Like Domain and the Cytokine Receptor Homologous Domain", GROWTH FACTORS, 1997, Vol. 14, No. 2-3, pages 117-130, XP002919519.

## Description

This application claims priority to U.S. Provisional Application Serial No. 60/083,575, filed on April 30, 1998.

The process by which blood cells grow, divide and differentiate in the bone marrow is called hematopoiests (Dexter, T. M., and Spooneer, E., Annu. Rev. Cell Biol., 3: 423, 1987). There are many different types of blood cells that belong to distinct cell lineages. Each of the various blood cell types arises from pluripotent stem cells that are able to undergo self-renewal, or give rise to progenitor cells that yield all of the different mature cell types. Three general classes of cells are produced *in vivo*: red blood cells (erythrocytes), platelets, and white blood cells (leukocytes), the vast majority of the latter being involved in host immune defense.

Proliferation and differentiation of hematopoietic precursor cells are regulated by a family of cytokines, including colony-stimulating factors (CSFs) and interleukins (Arai, K-I., et al, Annu. Rev. Biochem. 1990, 59:783-836). At least four cytokines are involved in production of neutrophils and macrophages, that is, interleukin-3 (IL-3), granulocyte/macrophage colony-stimulating factor (GM-CSF), granulocyte colony-stimulating factor (G-CSF) and macrophage-stimulating factor (M-CSF). Among them, G-CSF works specifically on cells restricted to the neutrophilie granulocyte lineage (Demetri, G. D., and Griffin, J. D., Blood, 1991, 78: 2791-2808). The principal biological effect of G-CSF *in vivo* is to increase the proliferation and differentiation of neutrophils from committed progenitors (Cohen, A. M., Proc. Natl. Acad. Sci. USA, 1987, 84: 2484-2488). G-CSF also potentiates the migration, survival and function of mature neutrophils, including increasing phagocytic activity and antimicrobial killing (Crawford, J., et al., N. Engl. J. Med., 1991, 325: 164-170, Moore, M.A.S., Annu. Rev. Immunol. 1991, 9: 159). This physiologic process serves as the foundation for critical host defense systems and occurs on a large scale *in vivo.*

The half-life of a commercial form of recombinant human G-CSF (Neupogen®, Amgen, Inc.) *in vivo* is only 3.5 hours, and it has to be administrated daily to maintain the threshold level of G-CSF required for stimulating neutrophil generation (Physician's Desk Reference, 53^{rd}, 1999, 532-537). The major side effects of recombinant human G-CSF ("rhG-CSF") therapy at higher dosage is bone pain, presumably due to the transient high level of rhG-CSF *in vivo* immediately following injection; however, this is less frequent in patients receiving lower doses of rhG-CSF.

Various means are under investigation to prolong the *in vivo* half-life of rhG-CSF, including conjugation with polyethylene glycol (PEG). However, a recent report indicates the PEG conjugates had considerably lower activity than the unmodified proteins with an inverse correlation between molecular weight of the PEG moieties conjugated to the protein and activity *in vitro.* See Bowen S., et al, Exp. Hemat., 1999, 27: 425-432. Moreover, data from animal studies indicates that the rhG-CSF conjugated with PEG extends the half-life to 1 to 3 days, but not beyond that.

In contrast to PEG conjugated rhG-CSF, the *in vivo* half-life of monoclonal antibodies ("mAbs") is around 2-3 weeks, dependent upon the antibody isotype. It is anticipated that a single injection of an agonist antibody against human G-CSF receptor will provide G-CSF-like activity for several weeks. Thus, patients with chemotherapy and severe chronic neutropenia would potentially benefit from less frequent hospital visits due to the prolonged biological activity of agonist mAbs. Additionally, sustained levels of the agonist antibody in the blood circulation would continue to stimulate the proliferation and differentiation of neutrophilic progenitor, therefore exhibit higher potency, resulting in lower dose usage and possibly fewer side effects than Neupogen® or other rhG-CSF derivatives.

Various actions of G-CSF are triggered by the binding of G-CSF, through its two discrete binding sites, to its receptors, forming a 1:2 ligand/receptors complex. The G-CSF receptor, expressed on the progenitor cells of neutrophlic granulocytes and on mature committed cells, belongs to the superfamily of cytokine/hematopoietic receptors. Although the majority of family members, including the receptors for the interleukins from interleukin-2 (IL-2) to IL-7 and granulocyte-macrophage colony-stimulating factor (GM-CSF), are activated through the formation of heteromeric complexes composing α, β, and sometimes even y subunits, G-CSF receptor protein, consisting of a single chain polypeptide, is believed to form a homodimeric complex upon ligand binding (Fukunaga, R., et al., J. Bio. Chem.,1990, 265: 14008).

Homodimerization of the G-CSF receptor has been shown to be essential for signal transduction (Wells, J. A., and Vos, A. M., Annu. Rev. Biochem.,1996, 65: 609). The G-CSF receptor does not contain an intrinsic protein kinase domain although tyrosine kinase activity seems to be essential to transduction of the G-CSF signal. The signal from G-CSF receptor activation through G-CSF induced receptor homodimerization is mediated by noncovalent binding of various tyrosine kinases, *e*.*g*. JAK1 and JAK2 (Barge, R. M. Y., et al, Blood, 1996, 87: 2148-2153), and thereafter the phosphorylation of transcription factors Stats such as Stat3 and Stat5 (Tian, S-S., et al, Blood, 1994, 84: 1760-1764; Watowich, S. S., et al., Annu. Rev Cell Dev. Biol., 1996,12: 91; Dong F., et al, J. Immunol., 1998, 161: 6503-6509). These tyrosine kinases play an essential role for G-CSF receptor phosphorylation and Stat activation in response to G-CSF (Tian S-S. et al blood, 1996, 88: 4435-4444; Shimoda, K.,et al., Blood, 1997,90: 597-604).

Except for G-CSF receptor, the functions of the receptors for erythropoietin ("EPO"), growth hormone ("GH"), prolactin receptor ("PRL") and thrombopoietin ("TPO") also appear to be triggered by ligand-induced receptor homodimerization, resulting in phosphorylation of a specific set of kinases (Youssoufian, H., et al. Blood, 1993, 81: 2223; Alexander, W.S., et al EMBO, 1995, 14: 5569; Heldin C.H., Cell, 1995, 83: 213). Therefore, the screening methods disclosed in the invention, which are used to screen for G-CSF receptor agonists based on their ability to cause signal transduction on homodimerization and proliferation of receptor-bearing cells.

Antagonist antibodies are disclosed in WO 95/21684 and by Layton et al., Growth Factors, 1997, vol 14, pp. 117-130. WO 97/12977 discloses peptides as agonists to the G-CSF receptor

The present invention relates to an agonist antibody or a binding fragment thereof which dimerizes the human G-CSF receptor wherein said dimerization stimulates cell proliferation and differentiation.

Thus, the invention relates to interactive agonist antibody molecules to the human G-CSF receptor, which, by binding to or interacting with such receptors, play the same biological roles as the ligands do. The invention includes agonistic antibody molecules capable of binding to, or interacting with two human G-CSF receptor proteins. These agonistic molecules include whole antibody molecules, both polyclonal and monoclonal, as well as modified or derived forms thereof, including immunoglobulin fragments like bivalent F(ab')₂ capable of exerting the same or a similar agonist effect as the native G-CSF. The agonist antibodies and fragments can be animal-derived, human-mouse chimeric, humanized, DeImmunised™ or fully from human.

By binding to two G-CSF receptors, G-CSF receptor agonist antibodies stimulate growth and/or differentiation of cells expressing the G-CSF receptor. This is accomplished by binding to the extracellular domain of the G-CSF receptor, and dimerizing the G-CSF receptor whereby activation of phosphorylation of kinases associated with the G-CSF receptor occurs. These cells expressing the G-CSF receptor generally comprise primitive stem/progenitor hematopoietic cells and thus the agonists will promote primitive hematopoietic cells to differentiate and/or proliferate leading to a repopulation of neutrophilic granulocyte lineage cells.

Another aspect of the invention relates to a method for screening for G-CSF receptor agonist antibodies, using an *in vitro* cell-based assay system. As described below, cells can be transfected with the G-CSF receptor, or the portion of the G-CSF receptor which is activated upon binding the agonist, and then the cells can be monitored for its proliferation in the presence of the agonist antibody molecule.

The invention also includes the use of such agonist antibodies, for both diagnostic purposes and therapeutic applications. The hybridomas producing exemplary agonist antibodies, designated mAb163-93 and mAb174-74-11, were deposited at the American Type Culture Collection, 10801 University Blvd., Manassas, VA 20110-2209 ("ATCC"), under Accession Nos. HB-12699 and HB-12700, respectively.
FIG. 1A shows that the proliferation of the parental mouse cell 32D-c123 is stimulated only by rmIL-3, but not by rhG-CSF (R&D Biosystems), as determined by an MTT assay.
FIG. 1B shows that after 32D-c123 was transfected with the full-length of the human G-CSF receptor, the proliferation of the transfectant D4 cells can be stimulated by rmIL-3 and rhG-CSF, separately, as determined by an MTT assay. FIG. 1C shows that the ³H-thymidine uptake by the transfectant D4 cells increases in a concentration-dependent manner when growing in the media containing rmIL-3 or rhG-CSF, but not the control mAb.

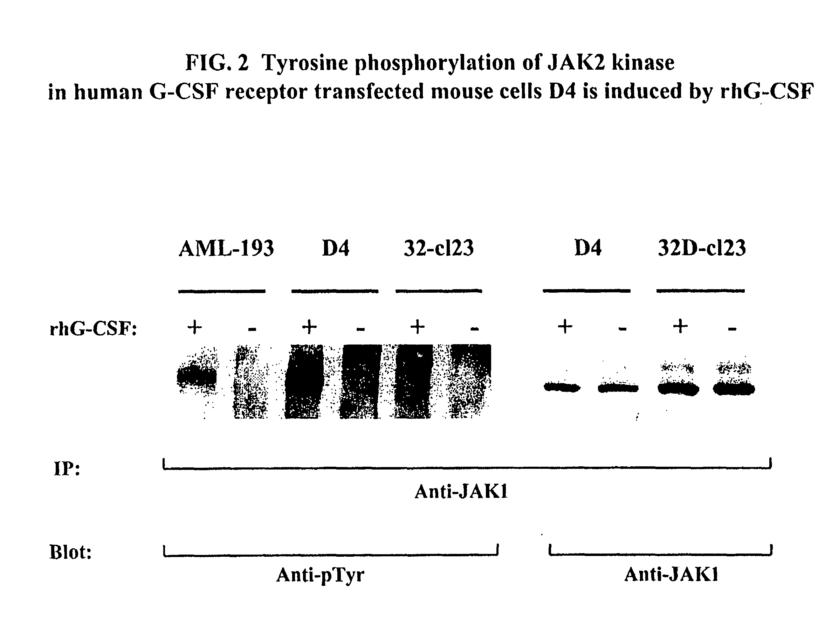

FIG. 2. shows tyrosine phosphorylation of JAK1 kinase in the full-length human G-CSF receptor transfected D4 cells induced by rhG-CSF. The same tyrosine phosphorylation of JAK1 kinase can not be detected in the parental cells 32D-c123, even in the presence of rhG-CSF (R&D Biosystems). As the positive control, the human G-CSF-responsive cells AML-193 (ATCC No. CRL-9589) expressing the endogenous human G-CSF receptor also shows that the tyrosine phosphorylation of JAK1 kinase is induced by stimulation of rhG-CSF. IP: Immunoprecipitation with the antibodies as indicated in the figure. Blot: detection with HRP-conjugated antibodies as indicated. Anti-pTyr: anti-phosphotyrosine antibody 4G10 (Upstate Biotechnology, Lake Placid, NY).
FIG. 3 shows the binding of mAb163-93 to G-CSF receptor/IgG4(Fc) chimeric protein by ELISA. The human G-CSF receptor/IgG4(Fc) was caught by goat anti-human IgG(Fc) antibody coated on the Immulon 2 plate. The binding of the mouse antibody mAb163-93 to the human G-CSF receptor/IgG4(Fc) chimeric protein was detected by binding of the goat anti-mouse IgG(Fc) antibody conjugated with horseradish peroxidase.
FIG. 4A shows that the binding percentage of mAb163-93, but not of the isotype-matched control mAb G3-519, to the full-length human G-CSF receptor transfected mouse cells D4 increases in the concentration-dependent manner by FACS analysis. FIG. 4B shows that the mAb163-93 specifically binds to mouse cells D4 expressing full-length human G-CSF receptor, but not to its parental cells 32D-c123 as indicated by cell bound percentages.
FIG. 5A and 5B show the proliferation of human G-CSF receptor transfected mouse cells D4 stimulated by various mouse monoclonal agonist antibodies, including mAb163-93 and mAb174-74-11, as measured by an MTT assay. The isotype-matched mAb G3-519 against HIV-gpt120, and rhG-CSF (R&D Biosystems) were set as the negative and positive controls.
FIG. 5C shows that a panel of monoclonal antibodies, including mAb163-93 and mAb1 74-74-11, can stimulate the proliferation of the human G-CSF receptor transfected mouse cells D4, as indicated by the increase of ³H-thymidine incorporation.
FIGS. 6A and 6B show tyrosine phosphorylation of kinase JAK2 (FIG. 6A) and transcriptional factor Stat3 (FIG. 6B) in the human G-CSF receptor transfected mouse cells D4 stimulated by cytokines rmIL-3, rhG-CSF, and the agonist antibody mAb163-93.
FIGS. 7A and 7B show a quantitative assay for stimulating granulocyte colony-formation from human bone marrow: FIG. 7A: rhG-CSF and mouse mAb 163-93 (The control mAb G3-519 is isotype-matched with mAb163-93) and FIG. 7B: other mouse agonist mAbs.
FIG. 8 shows neutrophilic granulocyte colony formation from human bone marrow stimulated by: 8A: isotype-matched control mAb G3-519; 8B: rhG-CSF (R&D Biosystems); 8C: monoclonal agonist antibody mAb163-93: 8D: Morphology of cells picked up from the colony in 8C, after cell staining.
FIG. 9 shows that the mouse mAb163-93 can stimulate granulocyte colony formation from chimpanzee bone marrow in a concentration-dependent manner.
FIG. 10 shows neutrophilic granulocyte colony formation from chimpanzee bone marrow stimulated by: 10A: isotype-matched monoclonal antibody G3-519 at the concentration of 50 nM: 10B: rhG-CSF (R&D Biosystems) at the concentration of 0.5 nM; 10C: monoclonal agonist antibody mAb163-93 at the concentration of 5 nM; 10D: Morphology of cells picked from the colony in C, after cell staining.

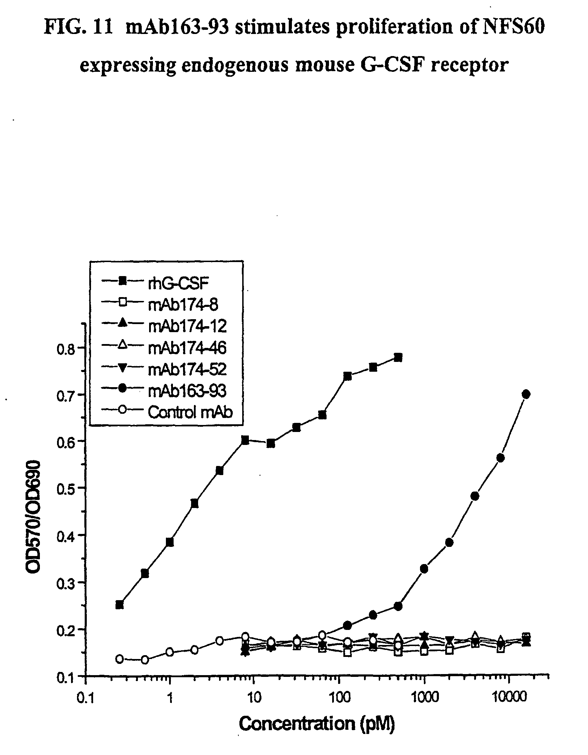

FIG. 11 shows that the agonist mAb163-93 against human G-CSF receptor stimulates the proliferation of mouse cells NFS60 expressing endogenous mouse G-CSF receptor.

SEQ ID NOS. 1 to 6 represent various primers used in cloning the G-CSF receptor.

SEQ ID NOS. 7 to 14 represent various primers used in cloning variable regions of two agonist antibodies of the invention.

SEQ ID NOS. 15 to 26 represent the amino acid sequences of the CDRs (both light and heavy chains) of two agonist antibodies of the invention.

SEQ ID NO. 27 represents the amino acid sequence of the extracellular domain of human G-CSF receptor.

### 1. Producing the Agonists of the Invention

The G-CSF receptor agonist antibodies described herein preferably target epitopes within the extracellular domain, of the G-CSF receptor. Exemplary agonists include the monoclonal antibodies produced by the hybridoma cell lines 163-93 and 174-74-11.

Monoclonal agonist antibodies of the invention can be produced by immunization and fusion (see Example 4 below), or from isolated lymphocytes using EBV transformation, or through human G-CSF receptor transfected insect or mammalian cells. The agonist antibodies are preferably chimeric, DeImmunised™, humanized or human antibodies for clinical use. Such antibodies can reduce immunogenicity and thus avoid human anti-mouse antibody (HAMA) response. It is preferable that the antibody be IgG4, IgG2, or other genetically mutated IgG or IgM which does not augment antibody-dependent cellular cytotoxicity (S.M. Canfield and S.L. Morrison, *J. Exp. Med*., 1991: 173: 1483-1491) and complement mediated cytolysis (Y.Xu et al., *J. Biol. Chem*., 1994: 269: 3468-3474; V.L. Pulito et al., *J. Immunol*., 1996; 156: 2840-2850).

Chimeric antibodies are produced by recombinant DNA processes well known in the art, and have animal variable regions and human constant regions. Humanized antibodies have a greater degree of human peptide sequences than do chimeric antibodies. In a humanized antibody, only the complementarity determining regions (CDRs) which are responsible for antigen binding and specificity are animal derived and have an amino acid sequence corresponding to the animal antibody, and substantially all of the remaining portions of the molecule (except, in some cases, small portions of the framework regions within the variable region) are human derived and correspond in amino acid sequence to a human antibody. See L. Riechmann et al., *Nature*, 1988; 332: 323-327; G. Winter, *United States Patent* No. 5,225,539; C. Queen et al., U.S. patent number 5,530,101.

DeImmunised™ antibodies are antibodies in which the potential T and B cell epitopes have been eliminated, as described in International Patent Application PCT/GB98/01473. Therefore, their immunogenicity in humans is expected to be substantially reduced when they are applied *in vivo*.

Human antibodies can be made by several different ways, including the use of human immunoglobulin expression libraries (Stratagene Corp., La Jolla, California) to produce fragments of human antibodies (V_{H}, V_{L}, Fv, Fd, Fab, or (Fab')₂), and using these fragments to construct whole human antibodies using techniques similar to those for producing chimeric antibodies. Human antibodies can also be produced in transgenic mice with a human immunoglobulin genome. Such mice are available from Abgenix, Inc., Fremont, California, and Medarex, Inc., Annandale, New Jersey.

All of the wholly and partially human antibodies are less immunogenic than wholly murine mAbs. Bivalent fragments, also suitable for use in the invention, are also less immunogenic. All these types of antibodies are therefore less likely to evoke an immunogenic response in humans. Consequently, they are better suited for *in vivo* administration in humans than Whole animal antibodies, especially when repeated or long-term administration is necessary, as is predicted for the agonist antibodies of the invention.

An alternative to administering antibodies to the patient is to generate agonist antibodies endogenously through gene therapy techniques. DNA sequences encoding the agonist antibodies or their fragments, derivatives, or analogs, can be delivered *in vivo* using standard vectors in gene therapy, including the adenovirus, AAV or a retrovirus, or by a non-vector delivery system. The sustained expression of the agonist antibodies, or their fragments may have additional advantages for clinical treatment of chronic neutropenia.

The extracellular domain of the human G-CSF receptor (used for generating antibodies against the human G-CSF receptor in the invention) can be generated using molecular recombinant DNA technology well known in the art. The extracellular domain extends from numbers 1-603 of the amino acid residues of mature human G-CSF receptor, starting from its N-terminus (SEQ ID NO:27). See, *e*.*g*., U.S. Patent Nos. 5,589,456; 5,422,248; 5,574,136. However, a portion of the extracellular domain of the human G-CSF receptor, in purified or partially purified form, can also be used as the immunogen.

This extracellular domain of the human G-CSF receptor can be directly used as the immunogen to immunize animals, *e*.*g*. mice, or it can first be fused with a carrier molecule to increase its immunogenicity prior to immimization. Suitable carrier molecules include peptides, *e*.*g*., Tag, Flag, leucine-zip, or a protein, *e*.*g*., glutathione-S-transferase (GST), alkaline phosphatase (AP), intein or a constant region of an immunoglobulin (as was used to make the agonist antibodies described below). The carrier molecule can be conjugated to the G-CSF by recombinant DNA techniques. La addition to enhancing the immunogenicity, such chimeric fusion proteins containing the extracellular domain of the G-CSF receptor can also facilitate the purification of the antigen, where affinity chromatography is used. The DNA fragments encoding these antigens containing the extracellular domain of the G-CSF receptor can be placed into expression vectors, which are then transfected into host cells such as *E. coli*, yeast, insect cells, and mammalian cells, including simian COS cells, Chinese hamster Ovary (CHO) cells, or myeloma cells. The antigens produced by this procedure can then be purified by techniques well known in the art.

Suitable immunogens include mutants of the native or wild-type G-CSF receptor extracellular domain, with substitutions, deletions or insertions, whether generated artificially or naturally occurring. Cells expressing G-CSF receptor or its analogs can also be used as the immunogens. Such cells include primary human cells and cell lines such as AML-193, human or mouse cells (or, optionally, insect cells using baculovirus as an expression vector) transfected with vectors for expressing the full-length, or a part of the G-CSF receptor, or a chimeric protein containing the extracellular domain of the G-CSF receptor (Talchashi, T., et al., J. Biol Chem. 1996, 271: 17555-17560).

To generate agonist antibodies against G-CSF receptor (polyclonal or monoclonal), the immunogens described herein can he used for immunizing rodents (*e*.*g*. mice, rats, hamsters and guinea pigs) or other mammals, including rabbits, goats, sheep, non-human primates, or transgenic mice expressing human immunoglobulins or severe combined immunodeficient (SCID) mice transplanted with human B lymphocytes or human bone marrow, through the procedures well known in the art. Hybridomas can be generated by conventional procedures by fusing B lymphocytes from the immunized animals with myeloma cells (*e*.*g*. Sp2/0 and NS0), as described by G. Köhler and C. Milstein (Nature, 1975, 256: 495-497). Antibodies against the G-CSF receptor can also be generated by screening recombinant single-chain Fv or Fab libraries from human B lymphocyte or human bone marrow in phage display systems (Hoogenboom and Winter, J. Mol. Biol., 1991, 227:381; Marks et al. J. Mol. Biol., 1991, 222: 581).

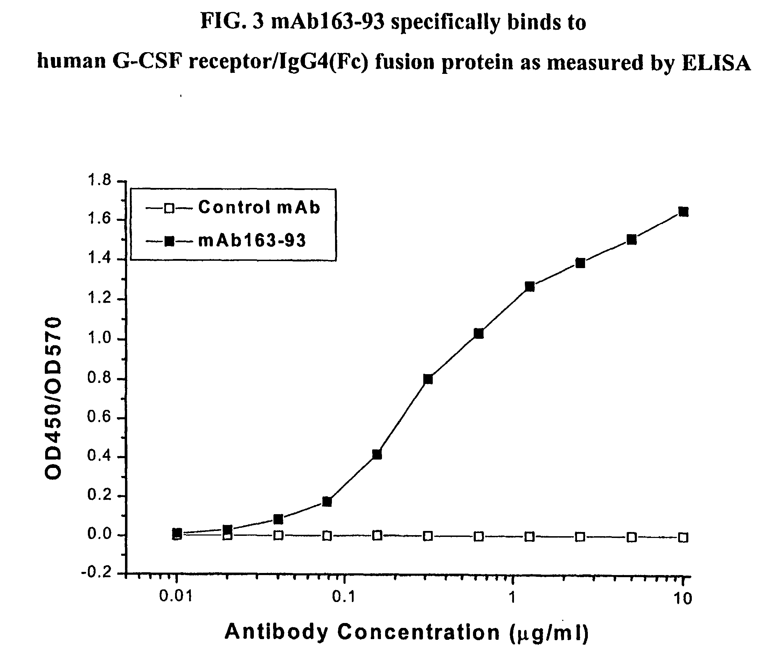

The selection of antibodies to the G-CSF receptor can be performed by conventional enzyme-linked immunosorbent assay (ELISA) method, such as direct and indirect sandwich assays, in which the antigen, or preferably a G-CSF receptor/IgG4(Fc) chimeric protein, is coated directly or indirectly, on the plates. Such binding of antibody mAb163-93 to the chimeric protein, as detected by ELISA, is shown in Fig.3. A competitive ELISA may be used to identify antibodies whose epitopes are close to, or overlay with those of the ligand (Current protocols in molecular biology, ed. Ausubel, F. M. et al, published by Wiley Interscience, 1996).

The rhG-CSF (R&D Biosystems, Minneapolis, MN) may be used in a competitive ELISA after the G-CSF receptor/IgG4(Fc) chimeric protein is, directly or indirectly, coated on the ELISA plates. The-binding of antibodies to the G-CSF receptor can be determined by addition of the second anti-mouse antibody, such as the goat anti-mouse antibody. The second anti-mouse antibody may be conjugated with various compounds and proteins for detection, including horseradish peroxidase.

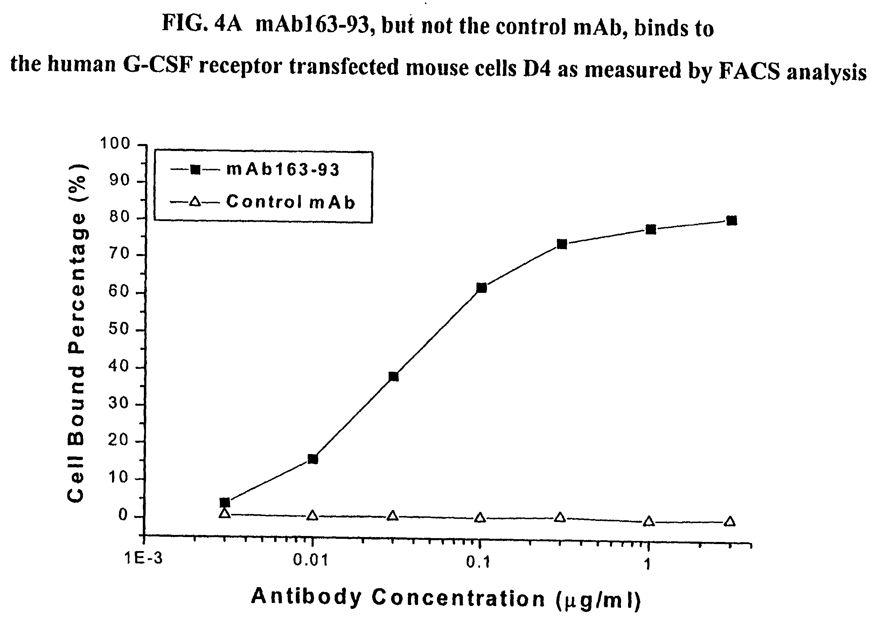

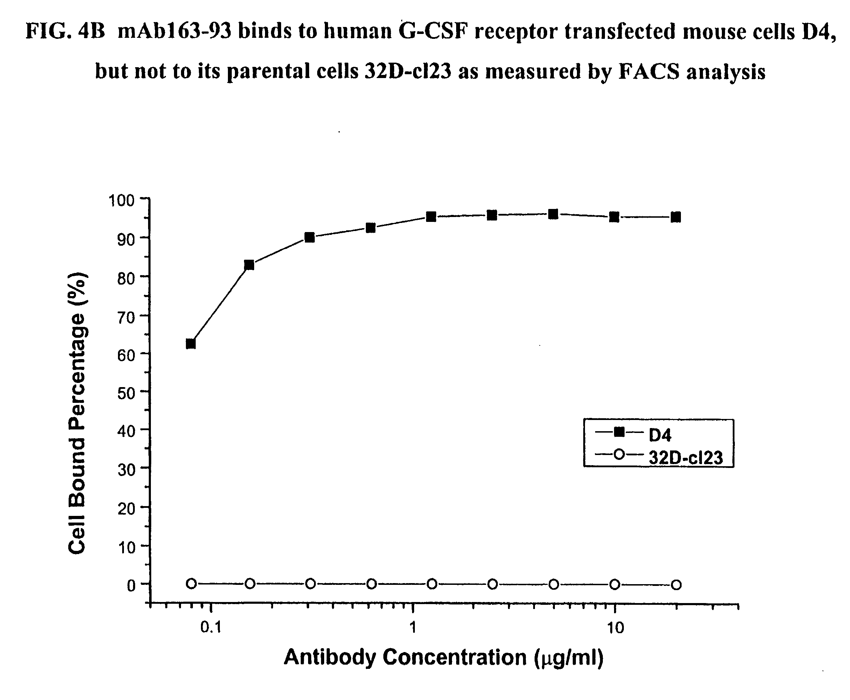

The binding specificity of the antibodies to the human G-CSF receptor expressed on the surface of cells, such as the transfectant mouse cells D4 described hereafter, can be determined by FACS analysis (Example 6). As shown in Fig. 4A, the murine monoclonal antibody mAb163-93 specifically binds to the mouse transfectant cells D4 expressing the human G-CSF receptor, but not the control mAb. Moreover, mAb163-93 specifically binds to the D4 cells expressing the human G-CSF receptor, but not to its parental cells 32D-c123·(Fig. 4B).

The screening method for G-CSF receptor agonist antibodies by *in vitro* cell-based biological function assays is also included in the invention. For large scale screening of agonists, one such approach involves constructing a G-CSF-responsive cell line such as NFS60 into which a construct of the cassette for expressing a reporter gene under the control of the promoter of G-CSF-responsive genes was integrated (Schindler, C. and Darnell, J. E., Annu. Rev. Biochem., 1995, 64: 621). The reporter used herein can be luciferase or galactosidase, green fluorescence protein or dihydrofolate reductase (Pelletier, J. N., et al., Proc. Natl. Acad. Sci. USA, 1998, 95: 4290). By measuring enzymatic activities or fluorescence densities in the cells after stimulation, the agonists against the G-CSF receptor can be selected by high-throughput screening.

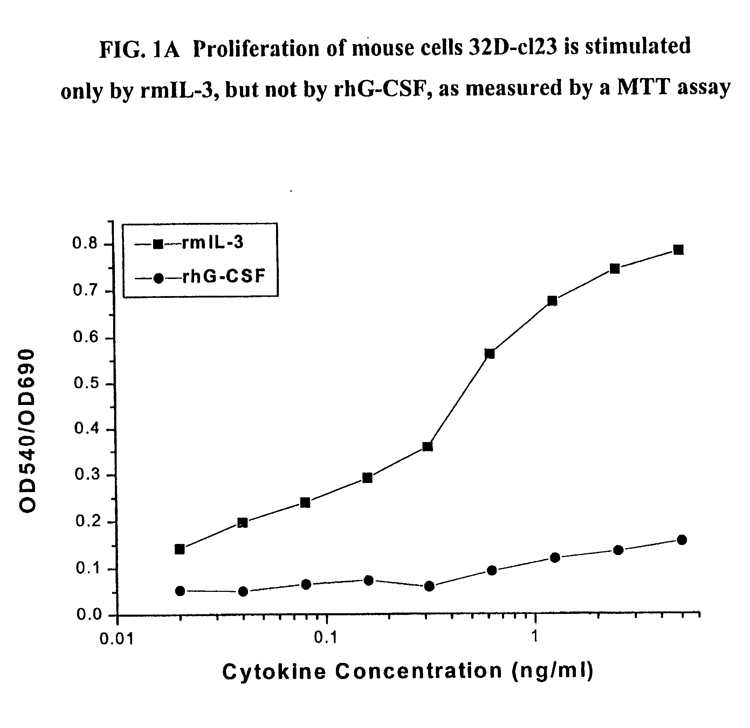

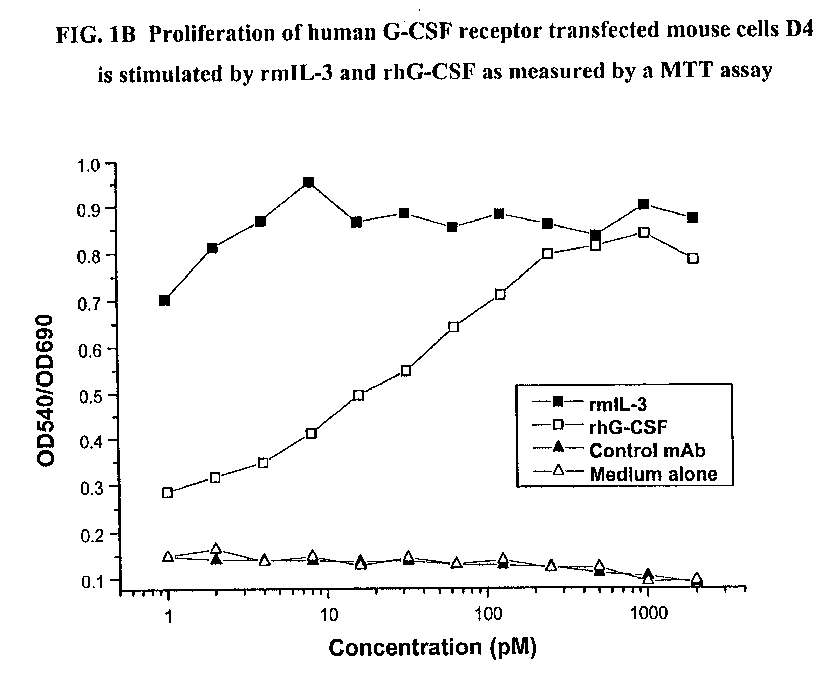

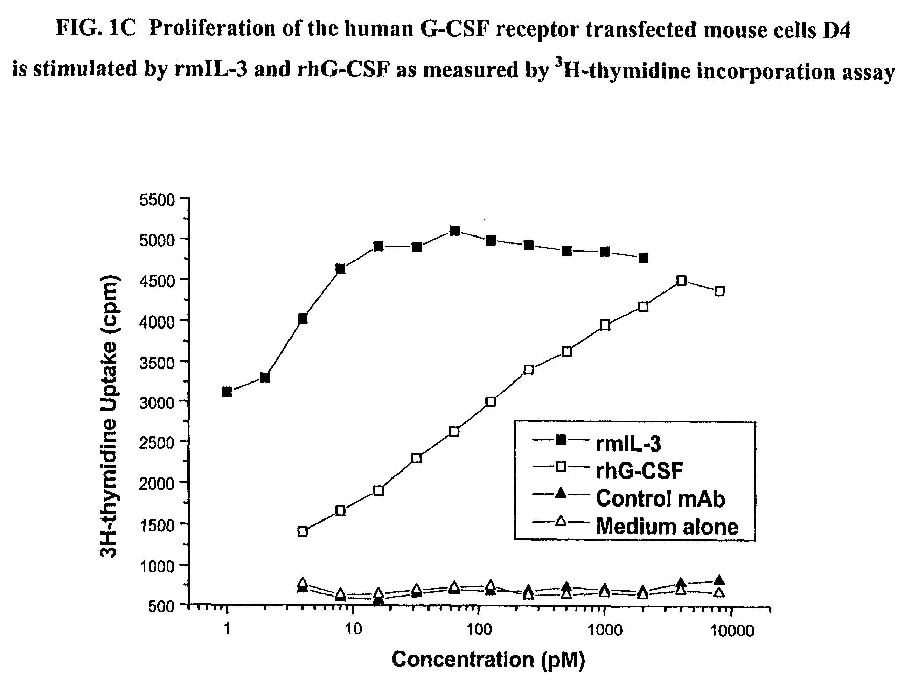

The *in vitro* cell-based biological function assays for agonists, including agonist antibodies, should also include an *in vitro* cell proliferation assay. As one of the preferred embodiments in the invention, a mouse cell line D4 expressing full-length human G-CSF receptor was constructed and used for screening agonist antibodies in large scale, which was combined with an MTT-based colorimetric assay. The colorimetric assay system using MTT is designed for the spectrophotometric quantification of cell growth in response to cytokines and their agonists without the use of radioacvtive isotopes. The parental mouse cell lines, such as BaF3 and FDC-P1, or preferably 32D-c123 in the Example 7 of the invention, are mIL-3 dependent and suitable as host cells for expression of the full-length of the human G-CSF receptor (Hapel, A. J., et al, Blood, 1984, 64: 786-790). After transfection with a vector in which the expression of full-length human G-CSF receptor is under the control of the constitutive hCMV promoter, and following selection, the transfectants, such as D4, also become responsive to G-CSF, as demonstrated by an MTT assay and a ³H-thymidine uptake assay, described in Example 7 below and shown in Fig. 1.

The phosphorylation of the tyrosine kinase JAK1 in the mouse transfectant cell line D4 is induced by rhG-CSF (R&D Biosystems), in the same manner as the human cell line AML-193 expressing endogenous human G-CSF receptor (Fig. 2). As described in Example 7, using the human G-CSF receptor transfected mouse cell line D4 combined with the MTT assay greatly facilitates the screening process for agonist antibodies. This screening method can also employ native G-CSF-dependent human cell lines, such as AML-193, mouse cell lines expressing human G-CSF, receptor mutants, or chimeric proteins including the extracellular domain of human G-CSF receptor fused with the intracellular domain of another cytokine receptor, such as the erythropoietin receptor (Goldsmith, M.A., et al, Proc. Natl. Acad. Sci. USA 1998, 95: 7006-7011) or the Fas receptor (Takahashi, T. et al., J. Biol Chem., 1996, 271: 17555-17560).

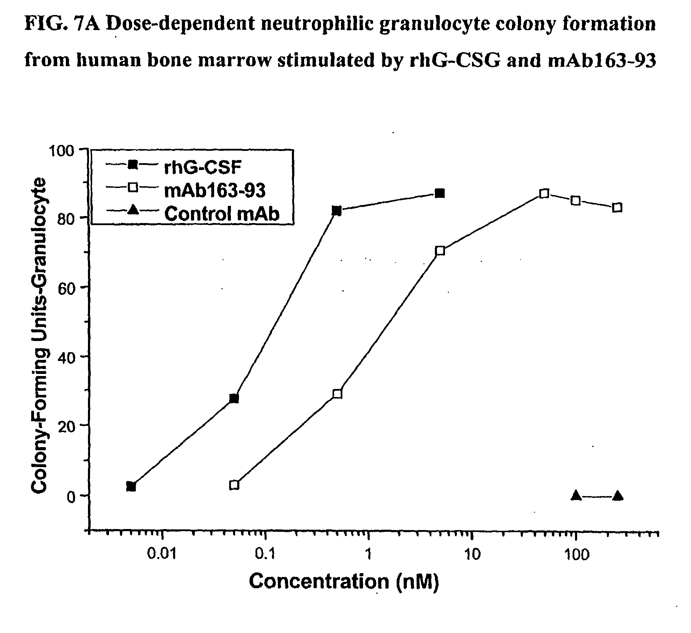

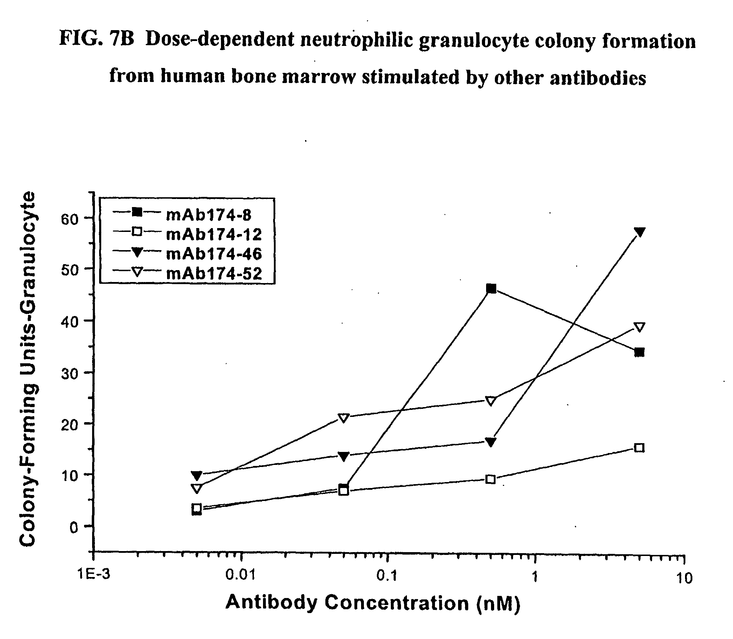

A granulocyte colony-forming assay using human bone marrow can also be used for screening agonist antibodies of the invention. Moreover, this assay can also be used for determining the potency, efficacy and specificity of agonist antibodies to stimulate the proliferation and differentiation of neutrophlic granulocytes from human bone marrow, and its species cross-reactivity to non-human primates. The results from this assay show that the agonist antibodies of the invention act as recombinant human G-CSF and specifically stimulate differentiation and proliferation of neutrophilic granulocytes from human bone marrow in a concentration-dependent manner. Furthermore, the agonist antibody mAb 163-93 shows efficacy in this assay essentially the same as that of rhG-CSF (Fig. 7). Further, the cells picked up from the colonies stimulated by the agonist mAb from human bone marrow display typical neutrophil morphology (Fig. 8D). It should be understood that rhG-CSF, after injection, is rapidly cleared from the circulation *in vivo,* primarily via the kidney, resulting in its short-duration pharmacological effects (Tanaka, H., et al, J. Pharmacol. Exp. Ther. 1989, 251: 1198-1203; Layton, J.E., et al, Blood, 1989, 74: 1303-1307). On the other hand, in the granulocyte colony-forming assay described in the invention rhG-CSF exhibits sustained activity to stimulate proliferation and differentiation of neutrophils from human bone marrow, compared with that *in vivo* due to lack of such clearance mechanism. It can be anticipated that the potency of the agonist antibody mAb163-93, due to its long half-life *in vivo*, to stimulate neutrophil proliferation and differentiation will be equal to, and even higher than that of rhG-CSF, as suggested by comparison of long-half Pegylated human growth hormone and human growth hormone (Clark, R., et al, J. Bio. Chem., 1996, 271: 21969-21977).

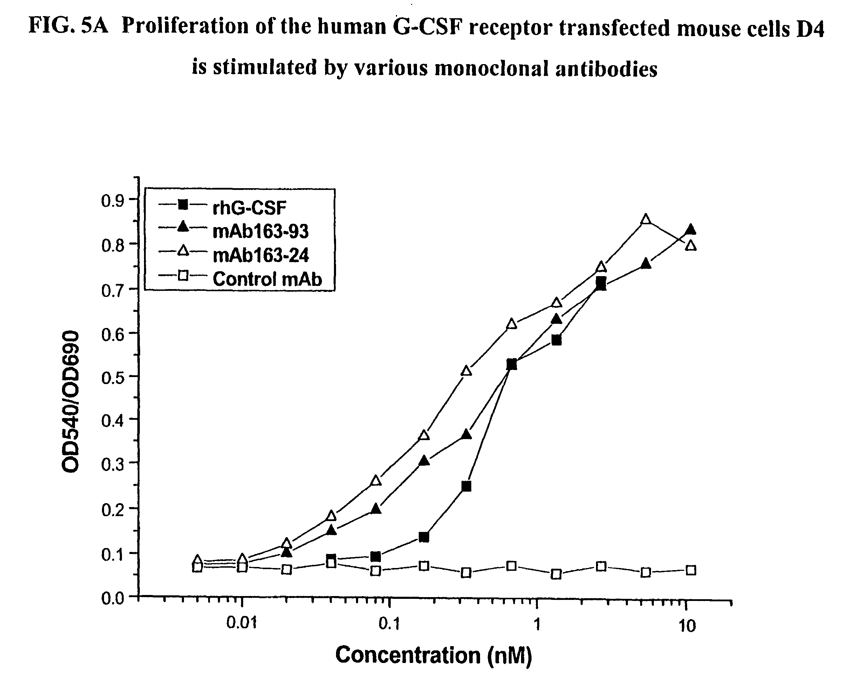

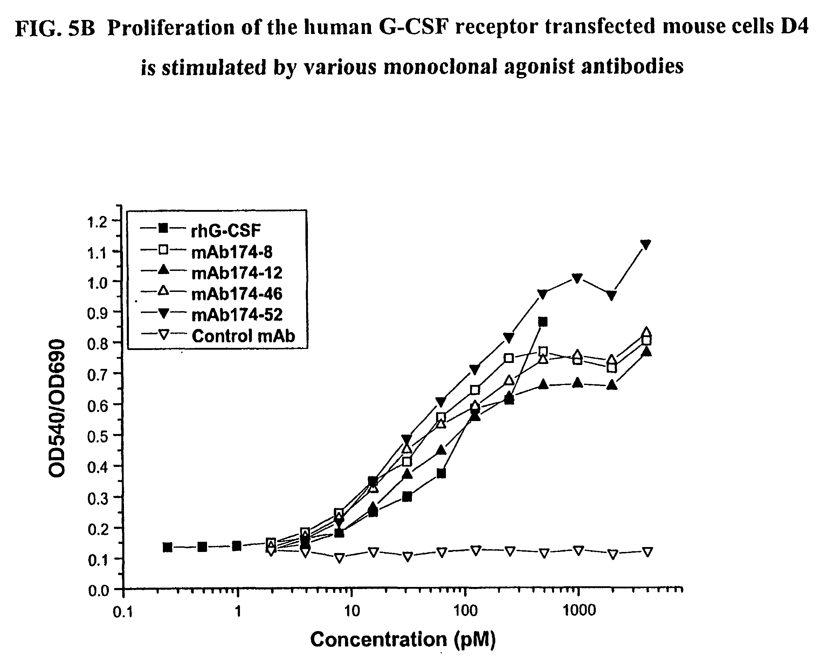

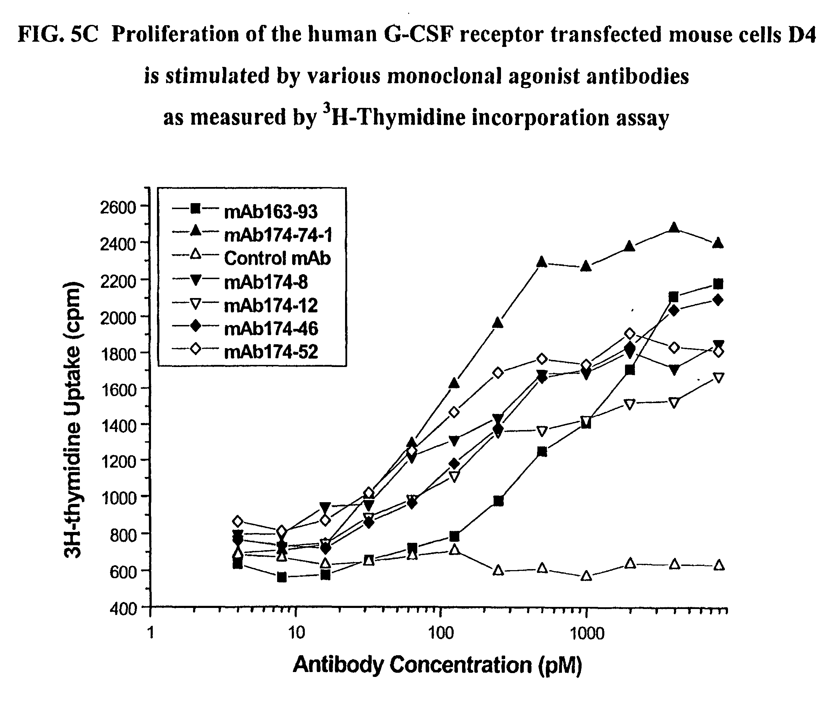

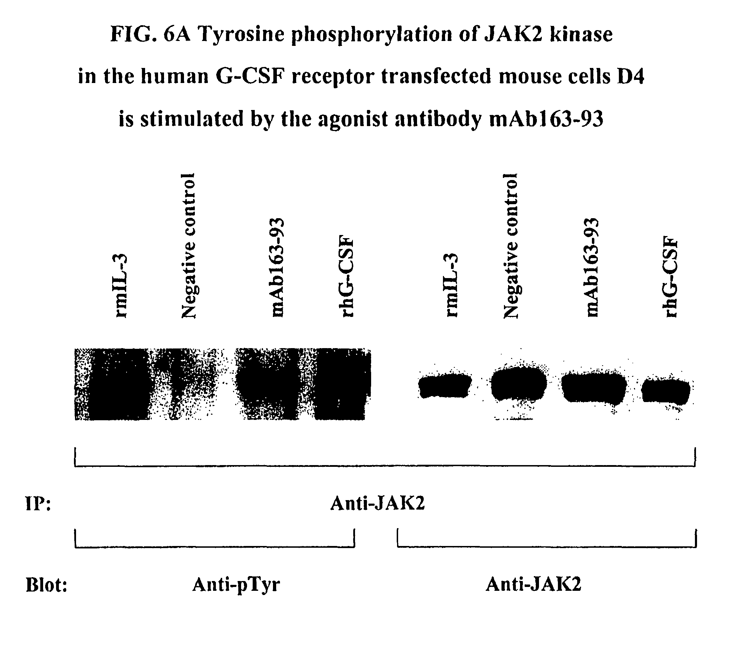

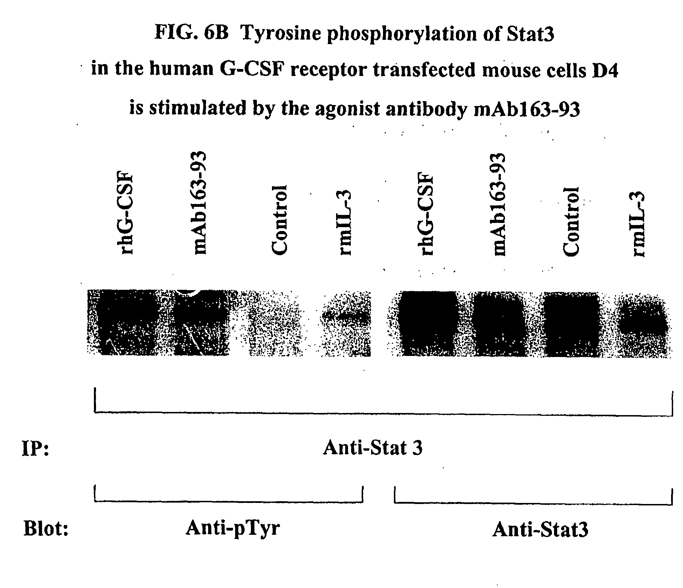

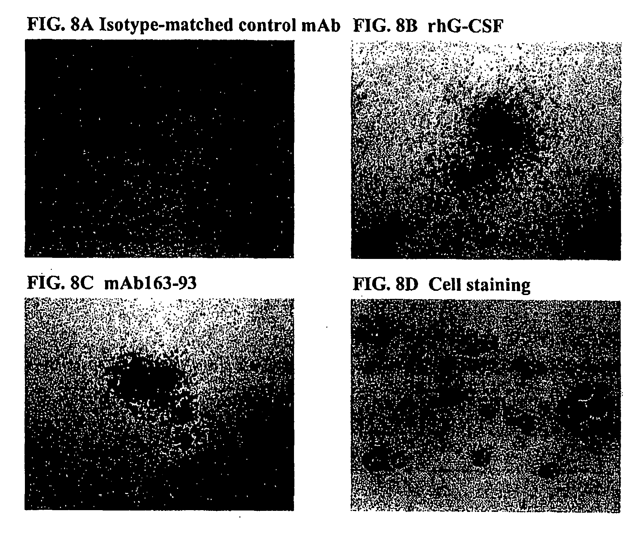

Using the foregoing screening techniques, a panel of monoclonal agonist antibodies, including mAb163-93 and mAb174-74-11, was generated against the human G-CSF receptor. The agonist antibody mAb163-93, acting as the' recombinant human G-CSF, activates G-CSF receptor, induces the tyrosine phosphorylation of JAK kinases (Fig. 6A, left hand panels where phosphorylation is detected by anti-pTyr antibody) and transcriptional factors (Fig. 6B, left hand panels where phosphorylation is detected by anti-pTyr antibody). Several agonist antibodies in the panel of antibodies generated in the invention were shown to stimulate the proliferation of G-CSF responsive cells *in vitro* (Fig. 5A-C). The mAb163-93 was shown to bind specifically to the G-CSF receptor on the cell surface (Figs. 4A and 4B). Moreover, these human G-CSF receptor agonist antibodies specifically stimulate neutrophilic granulocyte colony formation from human bone marrow, which is a further indication of their *in vivo* efficacy. (Figs. 7A, 7B and 8A-C). This is the first instance of monoclonal antibodies stimulating neutrophilic granulocyte colony formation from human bone marrow

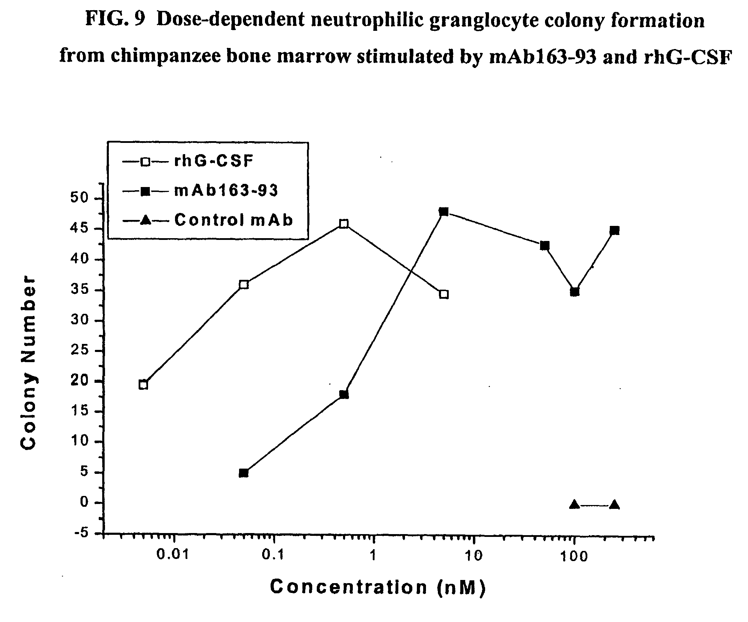

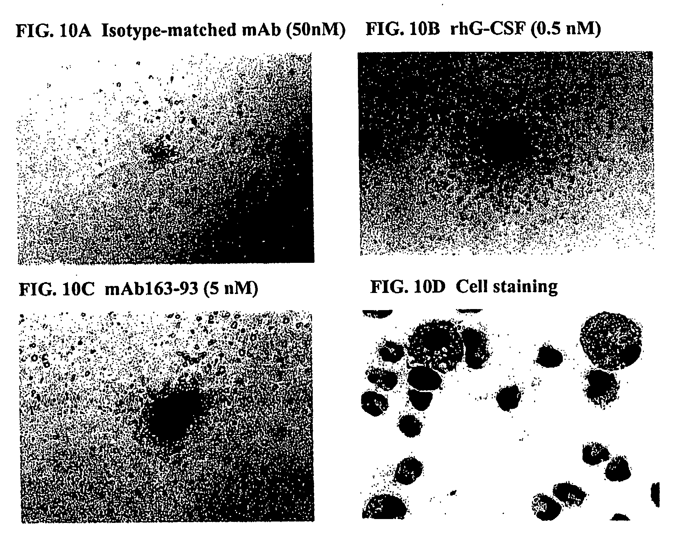

The species cross-reactivity of the human G-CSF receptor agonist antibodies was also determined using a granulocyte colony-forming assay. These human G-CSF receptor agonist antibodies, such as mAb163-93, were shown to specifically stimulate the proliferation and differentiation of neutrophilic granulocytes from various non-human primate bone marrows to varying degrees (Table 1 below). The number of granulocyte colony formation stimulated by the agonist antibody mAb163-93 from chimpanzee bone marrow increases in a concentration-dependent manner (Fig. 9). The results from cell staining show the specificity of this agonist mAb for stimulating the proliferation and differentiation of neutrophils from chimpanzee bone marrow (Fig. 10). This type of species crossreactivity assay can be used to select the appropriate animal model for preclinical studies.

**Table 1.**

| Neutrophilic granulocyte colony formation from non-human primate bone marrow | | |
|---|---|---|
| Non-human primate | rhG-CSF (0.5 nM) | mAb163-93 |
| Chimpanzee | 68 | 62 (5 nM) |
| Rhesus Monkey | 43 | 41 (50 nM) |
| Cynomolgus Monkey | 70 | 40 (50 nM) |
| Baboon | 36 | 3 (50 nM) |

The foregoing demonstrates that bivalent agonist antibodies are capable of activating the G-CSF receptor, *i*.*e*., they are capable of cross-linking the G-CSF receptors in a fashion that mimics the ability of G-CSF to form a complex and activate the receptor.

### 2. Using the Agonists of the Invention

Recombinant human G-CSF was among the first cytokines to be prepared by recombinant DNA technology and is successfully applied in therapy. This cytokine is widely used to reduce the incidence of infection associated with a variety of congenic and iatrogenic neutropenia. To date, five disease indications for rhG-CSF treatment have been approved by the United States FDA: (1) cancer patients receiving myelosupressive chemotherapy; (2) patients with Acute Myeloid Leukemia induction or consolidation chemotherapy; (3) cancer patients receiving bone marrow transplants; (4) cancer patients with peripheral blood progenitor cell collection and therapy; and (5) patients with severe chronic neutropenia (Physican's Desk Reference, 53^{rd} edition, 1999, 532-537). The unique functional specificity of the rhG-CSF on the proliferation and differentiation of the neutrophilic granulocyte lineage also makes it useful in other disease indications, including for HIV patients, patients with the systemic inflammatory response syndrome (SIRS) and sepsis, and patients with diabetic foot infection and other infectious diseases (Miles, S. A., et al, Blood 1990, 75: 2137-2142; Kuritzkes, D. R., et al, AIDS 1998, 12: 65-74; Weiss, M. et al, Bloob 1999, 93: 425-439; Lancet 1997, 350: 855-859; Deresinski, S. C., et al, Infect. Med. 1998, 15: 856-70).

The human G-CSF receptor antibodies disclosed herein, acting as rhG-CSF, activate the G-CSF receptor and stimulate the proliferation of G-CSF responsive cells through their specific binding to the G-CSF receptor on the cell surface, through the mechanism of inducing the tyrosine phosphorylation of JAK kinases and transcriptional factors. Furthermore, the human G-CSF receptor agonist antibodies specifically stimulate neutrophilic granulocyte colony formation from human bone marrow, as does the rhG-CSF. Therefore, the human G-CSF receptor agonist antibodies disclosed herein, are generally expected to be useful in all of the same therapeutic applications as rhG-CSF. Moreover, the longer half-life and *in vivo* stability of the agonist antibodies provides sigaificant Potential advantages over rhG-CSF fortherapeutic treatment.

The agonist antibodies of the invention can be administrated in an appropriate pharmaceutical formulation by a variety of routes, including, but not limited to, by intramuscular, intraperitoneal and subeutanous injection. The dosages can be determined by extrapolation from animal models and by routine experimentation during clinical trials.

These agonist antibodies of the invention are also useful for the affinity purification of G-CSF receptor from recombinant cell culture or from the natural source. General affinity purification techniques are well known in the art, and any of these may be used for this purpose.

The antibodies of the invention react immunologically with the soluble extracellular domain of the G-CSF receptor and cells expressing G-CSF receptor on their surface. Hence, the present invention also provides a method for immunologically detecting and determining existence of the G-CSF receptor in its soluble form, and/or on the cell surface, using immunological methods well known in the art. Normal, abnormal or mutated receptor structure or receptor expression can also be determined by using antibodies disclosed herein through immunoreactivity studies. The results can be useful for the diagnostic and treatment purposes.

### Example 1. Cloning of the extracellular portion of human G-CSF receptor protein:

The cloning of the G-CSF receptor protein was performed as follows. One ng of human bone marrow cDNA (Clontech, Palo Alto, CA) was used as the template in the PCR. It was added to 100 µl of a reaction mixture, which included the primers: AAG TGG TGC TAT GGC AAG GCT G (SEQ ID NO: 1); and CAC TCC AGC TGT GCC CAG GTC TT (SEQ ID NO: 2), at a final concentration of 500 nM. These primers are known to be homologous to the 5' and 3' ends of cDNA eocoding the extracellular portion of the human G-CSF recepto. The reaction conditions were as follows: 1 minute at 94°C; 30 secs. at 62°C; 3 minutes at 72°C; repeat for 40 cycles.

A DNA fragment resulting from the PCR (about 1.6 kb) was isolated from the agarose gel, according to the protocol from BIO101 Inc. (Vista, CA), and then inserted into a TA cloning vector (Invitrogen, Carlsbad, CA), yielding the recombinant plasmid pTI-11. The DNA sequence of this insert was determined by sequencing both strands of this insert using a DNA sequencing kit from United States Biochemical (Cleveland, Ohio). The DNA fragment encoding the extracellular portion of the human G-CSF receptor (using the sequence as defined by Fukunaga, R. et al., Proc. Nat'l Acad. Sci. USA, 1990, 87:8702) in pT1-11 was digested with EcoR1, and the ends were filled in by Klenow fragment. This was then inserted into the plasmid pFc1 containing IgG4(Fc) encoded cDNA, which was digested with Xba1. The ends were filled in by Klenow fragment, yielding the plasmid pFT1-9. The DNA fragment encoding the extracellular portion of human G-CSF receptor and IgG4(Fc) in pFT1-9 was digested with Asel and HincII, filled in by Klenow fragment, and then inserted into mammalian expression vecter pcDNA3 (Invitrogen). This vector was digested with EcoRV and HincII, and then filled in by Klenow fragment, yielding the plasmid pCGC23, in which the expression of the extracellular portion of human G-CSFR/IgG4(Fc) is under the control of the hCMV promoter.

### Example 2. Expression of the extracellular portion of hG-CSFR/IgG4(Fc) chimeric protein in mammalian cells:

NSO cells were transfected with linearized pCGC23 as follows. 4 x 10⁷ log-phase NSO cells were harvested and resuspended in 0.8 ml IMDM medium supplemented with 2% FBS. After incubation with 10 µg of linearized plasmid DNA for 10 minutes on ice, the cell mixture was subjected to electroporation at 200 volts and 960 µF, using a BioRad apparatus. After 20 minutes on ice, 100 µl of the diluted cell suspension was added to each well of about twenty 96-well plates. Two days later, another 100 µl of the same IMDM medium but containing G418 (Gibco BRL, Gaithersburg, MD) was added into each well to make the final concentration of G418 at 0.8mg/ml. After 10 days, culture supernatants were withdrawn for screening for the expression of the extracellular portion of human G-CSF receptor/IgG4(Fc) fusion protein by ELISA, as follows.

The wells of Immulon 2 plates (Dynatech Laboratories, Chantilly, VA) were coated with 50 µl of anti-human IgG(Fc) antibody at a concentration of 1 µg/ml, and incubated overnight at room temperature. After the coating solution was removed by flicking the plates, 200 µl of BLOTTO (5% non-fat dry milk in PBS) were added to each well at room temperature to block non-specific bindings. One hour later, the wells were washed with PBST buffer (PBS containing 0.05% Tween 20). Fifty microliters of culture supernatant from each well in the transfection plates were collected and mixed with 50 µl of BLOTTO, and then added to individual wells of the microplates. After one hour of incubation at room temperature, the wells were washed with PBST. The bound extracellular human G-CSF receptor/IgG4(Fc) fusion protein was detected by reaction with horseradish peroxidase conjugated with goat anti-human IgG (H+L) (Jackson ImmunoResearch Laboratories, West Grove, PA), which was diluted at 1:2000 in BLOTTO. Peroxidase substrate solution containing 0.1% 3,3' 5,5' tetramethyl benzidine (Sigma, St. Louis, MO) and 0.0003% hydrogen peroxide (Sigma) were added to each well for color development and left for 30 minutes. The reaction was terminated by addition of 50 µl of 0.2 M H₂SO₄ per well. The OD₄₅₀₋₅₇₀ reading of the reaction mixture was measured with a BioTek ELISA Reader (BioTek Instruments, Winooski, VT).

The transfectants with high OD₄₅₀₋₅₇₀ reading were picked up and single cell cloning was performed by the limiting dilution method. The same ELISA and detection as described in the foregoing paragraph were done to further identify the high producer cell line expressing the fusion protein comprising the extracellular portion of the human G-CSF receptor and the IgG4(Fc) chimeric protein.

### Example 3. Purification of the extracellular portion of human G-CSFR/IgG4(Fc) chimeric protein:

One liter of the culture supernatant from the transfectant cells expressing the extracellular portion of the human G-CSFR/IgG(Fc) chimeric protein was collected and the chimeric protein was purified from the supernatant by Prosep-A affinity chromatography, according to the manufacturer's instruction (Bioprecessing Inc., Princeton, NJ). The protein was further purified on a goat anti-human IgG(Fc) affinity column. The purity of this chimeric protein was determined by both SDS-PAGE and immunoblot.

### Example 4: Hybridoma generation:

BALB/c mice (Harlan, Houston, TX) were injected subcutaneously with 50 µg of the purified fusion protein consisting of the extracellular portion of human G-CSF receptor and IgG4(Fc) in complete Freund's adjuvant (Difico Laboratories, Detroit, MI) and in 200 µl of phosphate-buffered saline (PBS, pH7.4). The mice were boosted after 2 and 4 weeks with the same amount of the fusion protein in incomplete Freund's adjuvant. Then two weeks later and three days prior to sacrifice, the mice were given a final boost i.p. Their spleen cells were fused with Sp2/0 myeloma cells. 5 x 10⁸ of the Sp2/0 and 5 x 10⁸ spleen cells were fused in a medium containing 50% polyethylene glycol (MW 1450) (Kodak, Rochester, NY) and 5% dimethylsulfoxide (Sigma, St, Louis, MO). The cells were then adjusted to the concentration of 5 x 10⁴ spleen cells per 200 µl suspension in Iscove medium (Gibco BRL, Gaithersburg, MD), supplemented with 5% FBS, 100 units/ml of penicillin, 100 µg/ml of Streptomycin, 0.1 mM hypoxanthine, 0.4 µM aminopterin, and 16 µM thymidine. Two hundred microliters of the cell suspension were added to each well of one hundred microplates. After about ten days, culture supernatants were withdrawn for screening by using *in vitro* cell proliferation assay as described in Example 7.

### Example 5. Cloning of the cDNA encoding full-length human G-CSF receptor:

The total RNA from the human cell line AML-193 (ATCC catalog No. CRL-9589) was prepared by the Ultraspec-3 RNA isolation kit, according to the manufacturer's procedure (Biotex Laboratories Inc., Houston, TX). Ten micrograms of the total RNA from the AML-193 cell line were used as the template for synthesis of the first strand of cDNA in the reverse transcription reaction, according to the manufacturer's protocol (Gibco BRL, Gaithersberg, MD). To amplify the cDNA enconing the C-terminal half of the human G-CSF receptor, PCR was conducted in 50 µl of reaction mixture containig two primers: Nhe1: CCC CCC CAG CGC TAG CAA TAG CAA CAA GAC CTG GAG G (SEQ ID NO: 3); and R10: GGA ATT CCT AGA AGC TCC CCA GCG CCT CC (SEQ ID NO: 4), using the first strand of cDNA obtained as the template. The reaction conditions were as follows: 94°C for 1 minute; 60°C for 1 minute, and 72°C for 3 minutes for 40 cycles. The PCR product was cloned into the cloning vector pUC19 digested with Sma1, yielding the plasmid pC3. To create a new enzymatic cleavage site Nhe1 at the end of the cDNA fragment encoding the N-terminal half of the human G-CSF receptor, two primers were used in the PCR reaction, using the plasmid pCGC23 DNA as the template. These primers were T7P: AAT ACG ACT CAC TAT AG (SEQ ID NO: 5); and Nhe2: AGG TCT TGT TGC TAT TGC TAG CGC TGG GGG GGC CCA GG (SEQ ID NO: 6). The DNA fragment encoding N-terminal half of human G-CSF receptor from this PCR reaction was cloned into the vector pCR-Blunt (Invitrogen, carlsbad, CA), yielding the plasmid pB12. To assemble the full length of human G-CSF receptor DNA, the DNA fragment of the G-CSF receptor N-terminal half from the plasmid pB12 was inserted into the plasmid pC3 digested with Nhe1 and HincII, yielding the plasmid pCB1. The cDNA fragment encoding full-length of human G-CSF receptor was inserted into the mammalian expression plasmid pcDNA3 (Invitrogen, Carlsbad, CA), yielding the plasmid pCGF4.

### Example 6. Establishing and characterization of G-CSF-dependent mouse and human cell lines:

The human full-length G-CSF receptor expressing plasmid pCGF4, after linearizing with BspC1 digestion, was transfected into mouse cell line 32D-c123, or human cell line TF-1 (ATCC, VA) by electroporation as described above in Example 2. The transfectants were selected by growth in the RPMI 1640 supplied with 10% FBS, G418 at 0.8 mg/ml and mIL-3 at 1 ng/ml, or human GM-CSF at 1 ng/ml (R&D Systems, Minneapollis, MN), and further selected by an MTT assay using RPMI medium with 10% FBS, G418 at 0.6 mg/ml and rhG-CSF at I ng/ml (R&D Systems) as described in Example 7. The transfectants whose growth was stimulated by human G-CSF were further subjected to single cell cloning by the limiting dilution method.

The proliferation dependence upon human G-CSF of both the human and mouse transfectants, which contain the full-length human G-CSF receptor expression plasmid, was determined by growing these transfectants in the presence or in the absence of human G-CSF, human GM-CSF or mouse IL-3, as described in Example 7. The proliferation dependence of the transfectants upon these cytokines was monitored using the MTT assay and the ³H-thymidine uptake assay as described in Example 7.

The transfectants expressing human G-CSFR on the cell membrane surface were confirmed by FACS analysis. After washing with PBS plus 1% BSA, 50 l of the purified monoclonal antibody was added to the transfectant cells at a final concentration of 5 µg/ml. The cell mixtures were incubated on ice for 30 minutes and shaken every 15 minutes. After washing with cold PBS three times, goat anti-mouse IgG[F(ab')₂] conjugated with FITC was added at a 1:50 dilution to the transfectant cells and incubated for 30 minutes on ice. After washing three times with cold PBS, the cells were fixed with 1 % paraformaldehyde overnight. The cell binding percentage of transfectant cells with these mAbs was analyzed by FACS analysis.

### Example 7. In vitro cell proliferation assays:

The MTT-based colorimetric assay (te Boekhorst P.A., et al., Leukemia 1993, 7:1637-44) was used to screen and determine the ability of agonist antibodies to stimulate the proliferation of human G-CSF receptor transfected mouse or human cell lines. The transfectant cells pre-growing in RPMI medium containing 10% FBS and 1 ng/ml of mIL-3 were washed with RPMI with 10% FBS three times to remove mIL-3, then plated at 2-5 x 10⁴/well in RPMI with 10% FBS. The supernatants from the hybriboma plates, or purified antibodies, were added into each well. After three days of incubation, ten microliters of MTT (2.5 mg/ml in PBS, Boehringer-Maimheim Biochemical) was added into each well. After six hours of incubation, 100 µl of solubilizing solution containing 10% SDS and 0.01 N HCl were added to lyse cells, and the plates were incubated overnight. The proliferation of these G-CSF-dependent cells stimulated by the agonist antibodies can be monitored by reading at OD₅₄₀₋₆₉₀. In the MTT assay to determine the agonist activities of purified antibodies, the rhG-CSF and antibodies were diluted in series of 2-fold dilutions in duplicate or in triplicate.

The ability of these agonist antibodies to stimulate hG-CSF receptor transfectant cells can also be determined using a ³H-thymidine uptake assay. After washing three times with cytokine-free medium containing 10% FBS, 1-2 x 10⁴ transfectant cells (50 µl/well) were mixed with various concentrations of mIL-3, rhG-CSF (R&D Biosystems), hybridoma culture supernatants or purified antibodies in 96-well plates containing RPMI 1640 and 10% dialysed FBS. One µCi of ³H-thymidine (specific activity: 6.7 Ci/mmol, New England Nuclear) mixed with 50 µl of the same medium was then added to each well. After 48 hours incubation, the cells were harvested by a cell harvester (Skatron, VA) and ³H-thymidine uptake in triplicate was measured by a liquid scintillation analyser (Packard, IL).

### Example 8. Purification of human G-CSF receptor agonist monoclonal antibodies:

The antibodies generated from the hybridomas were purified by Prosep-A affinity chromatography, according to the manufacturer's instruction (Bioprocessing Inc., Princeton, NJ). The purity of the human G-CSFR agonist antibodies was checked using SDS-PAGE and Western blot. Two of the Mabs purified were designed as mAb163-93 and mAb174-24-11.

### Example 9. Bone marrow colony-forming assay:

About 10 ml of human bone marrow cells from healthy volunteers were collected and subjected to Ficoll-Paque separation, according to the standard method. The cells in the interface were carefully harvested with Pasteur pipetting, suspended with three volumes of IMDM and 2% FBS, and then centrifuged for 5 minutes at 400g. Use of this procedure gives a final marrow cell suspension that is enriched 2-4 fold in the content of primitive cells, because the more mature and denser myeloid cells are removed with the red blood cells. To determine the specificity of these human G-CSFR agonist antibodies, a granulocyte colony forming assay is performed according to the protocol provided by the manufacturer (StemCell Technologies Inc., Vancouver, Canada). To quantitatively measure the potency and efficacy of agonist antibodies to stimulate neutrophlic granulocyte colony formation from human or chimpanzee bone marrows, a series of different concentrations of agonist antibodies or rhG-CSF was applied in duplicate in this assay. As shown in Figs. 7 and 9, the agonist antibody mAb163-93, like rhG-CSF (R&D Biosystems), stimulates the neutrophilic granulocyte colony formation from human and chimpanzee bone marrow in a concentration-dependent manner. The results from cell staining show the specificity of this agonist mAb to stimulate the proliferation and differentiation of neutrophils from human and chimpanzee bone marrow (Figs. 8 and 10).

### Example 10. Tyrosine phosphorylation assay:

Tyrosine phosphorylation induced by cytokines and the agonist antibodies were analyzed as follows. About 2 x 10⁷ cells in log-phase was collected and starved in serum-free RPMI medium for four hours after washing three times with serum-free medium. The starved cells were stimulated with mIL-3, rhG-CSF (R&D Biosystems) or the agonist mAb at a final concentration of 2.6 nM for 15 minutes, and then harvested by centrifugation. The cells were lysed in 0.5 ml of lysis buffer (50 mN Tris.HCl, pH 7.5 / 150 mM NaCl / 1% (vol/vol) Triton X-100 / 1 mM EDTA with the addition of 1 mM Na₃VO₄, 1 µM pepstatin, 50 µM 3,4,dichloroisocpunmarin, I mM phenymethylsulfonyl fluoride, 1 mM 1, 10-phenanthroline, leupeptin (10 µg/ml) and aprotonin (10 µg/ml). After incubation on ice for 30 minutes, the lysates were cleared by centrifugation for 15 minutes at 14,000 rpm. For immunoprecipitation, rabbit polyclonal antibodies against JAK1, JAK2, or Stat3 (Upstate Biotechnology, Lake Placid, NY) were added into the clear lysates and incubated for two hours at 4°C. Then 50 µl of protein A beads (Gibco BRL) were added into each lysate and incubation was continued at 4°C for two hours. Following incubation, the beads were washed three times with the lysis buffer and suspended in 35 µl of Laemmli's sample buffer (62.5 mM Tris: pH 7.6, 2% SDS, 10% glycerol, and 5% 2-mercaptoethanol). The suspension was heated at 95°C for 5 minutes and electrophoresed on 4%-7.5% SDS-PAGE. After blotting, the blocked filters were incubated with HRP-conjugated mouse monoclonal anti-phosphotyrosine antibody 4G10 (Upstate Biotechnology) overnight at 4°C, according to the protocol from the manufacturer. After washing with PBS and PBS with 0.05% Tween 20, the filters were detected with SuperSignal Substrate kit (Pierce. Rockford, IL), according to the manufacturer's instruction. The nitrocellulose filters were reprobed with the antibodies and a second antibody conjugated with the horseradish peroxidase (HRP) as indicated.

### Example 11. Cloning and analyzing DNA fragments encoding variable regions of agonist antibodies from hybridoma cells:

Total RNA was prepared from hybridoma cells producing agonist antibodies, and used as the templates in the RT-PCR reaction as described in Example 1. The primers used in these PCR reactions are listed in SEQ ID NOS: 7 to 14 below. The DNA fragments generated from PCR reactions were cloned into a cloning vector pCR-Blunt (Invitrogen), then analyzed using automatic DNA sequencer Genetic Analyzer 310 (PE Applied Biosystems, Foster City, CA) according to the manufacturer's instruction. The individual recombinant plasmids from two separate RT-PCR reactions were analyzed to confirm the DNA sequences of the heavy and light chain variable regions were from the agonist antibodies.
The DNA sequences of primers used for cloning variable regions:
Primers for cloning mAb163-93 variable regions:
   For light chain:
   For heavy chain:
Primers for cloning mAb174-74-11:
   For light chain:
   For heavy chain:

The complementarity determining regions of the variable regions of these antibodies were shown to have the following sequences:
mAb163-93 (IgGl subclass), variable region heavy chain CDR sequences:
mAb163-93 variable region light chain CDR sequences:
mAb174-74-11 (IgG2a subclass) variable region heavy chain CDR sequences:
mAb174-74-11 variable region light chain CDR sequences:

It should be understood that the terms, expressions, examples, and embodiments described above are exemplary only and not limiting, that the scope of the invention is defined in the claims which follow, and includes all equivalents of the inventions set forth in the claims.

## Claims

1. An agonist antibody or a binding fragment thereof which specifically binds to or interacts with human G-CSF receptor and dimerizes it, wherein said dimerization stimulates cell proliferation and differentiation.

2. The agonist antibody or a binding fragment thereof of claim 1 which stimulates proliferation and differentiation of neutrophils or their progenitor cells.

3. The agonist antibody or a binding fragment thereof of claim 1 or 2 which interacts with the extracellular portion of human G-CSF receptor.

4. The agonist antibody or binding fragment thereof of any one of claims 1 to 3,
**characterized in that** the immunogen used to generate said antibody or binding fragment thereof comprises native human G-CSF or a mutant thereof with substitutions, insertions or deletions, provided that the resulting antibody or binding fragment thereof binds the human G-CSF receptor.

5. The agonist antibody or a binding fragment thereof of any one of claims 1 to 4 which interacts at an epitope between amino acid residues 1-603 (SEQ ID NO.: 27) of the G-CSF receptor.

6. The agonist antibody or a binding fragment thereof of any one of claims 1 to 5 which is a monoclonal antibody or binding fragment thereof such as F(ab)'₂.

7. The agonist antibody or a binding fragment thereof of any one of claims 1 to 6, wherein the CDRs of the heavy chain variable region of the agonist antibody include the following amino acid sequences: and wherein the CDRs of the light chain variable region include the following amino acid sequences: or

8. The agonist antibody or a binding fragment thereof of any one of claims 1 to 6, wherein the CDRs of the heavy chain variable region of the agonist antibody include the following amino acid sequences: and wherein the CDRs of the light chain variable region of the agonist antibody include the following amino acid sequences: or

9. The agonist antibody or a binding fragment thereof of any one of claims 1 to 6 which binds to the same epitope as the agonist antibody of claim 7 or 8.

10. The agonist antibody or a binding fragment thereof of any one of claims 1 to 9, wherein the antibody or a binding fragment thereof stimulates the proliferation of human or mouse cells expressing the human G-CSF receptor as determined in an *in vitro* proliferation assay.

11. A hybridoma cell line that produces the agonist monoclonal antibody or binding fragment thereof as defined in any one of claims 6 to 10.

12. A DNA sequence encoding the monoclonal antibody or a binding fragment thereof as defined in any one of claims 1 to 10.

13. A gene delivery system including the DNA sequence of claim 12.

14. The gene delivery system of claim 13 including the DNA sequences encoding the amino acid sequences shown in SEQ ID NO.: 15 through 20 or SEQ ID NO.: 21 through 26, or encoding a binding fragment thereof as defined in any one of claims 1 to 6.

15. The gene delivery system of claim 13 or 14, wherein the delivery system includes a viral vector, a plasmid, or a non-vector delivery system.

16. Use of the agonist molecule of any one of claims 1 to 10 for the preparation of a pharmaceutical composition for treating neutropenia.

17. A method of high throughput screening for an antibody having G-CSF agonist activity comprising constructing a G-CSF-responsive cell line, such as NFS60, with a construct capable of expressing a reporter gene, such as luciferase, β-dalactosidase, green fluorescence protein or dihydrofolate reductase, under the control of a G-CSF-responsive promoter and measuring enzymatic activities or fluorescence densities in the cells after stimulation by the antibody to be tested.

18. The method of claim 17, wherein the assay is used in combination with a colorimetric assay system such as measuring the reduction of 3-(4,5-dimethylthiazol-2-yl)-2,5-diphenyl-tetrazolium bromide (MTT) or an uptake assay using cells expressing G-CSF receptor.

19. The method of claim 17, wherein the construct includes the sequence of the extracellular domain of the G-CSF receptor.

20. An in vitro method for detecting human G-CSF receptor immunologically by means of an antibody as defined in any one of claims 1 to 10.

21. An in vitro method for immunological detection of a cell expressing human G-CSF receptor on the cell surface by means of an antibody as defined in any one of claims 1 to 10.

22. An in vitro method for detecting and determining a soluble human G-CSF receptor immunologically by means of an antibody as defined in any one of claims 1 to 10.

## Patentansprüche

1. Agonist-Antikörper oder ein bindendes Fragment davon, der oder das spezifisch bindet an den oder interagiert mit dem menschlichen G-CSF Rezeptor und ihn dimerisiert, wobei die Dimerisierung die Zellproliferation und -differenzierung anregt.

2. Agonist-Antikörper oder ein bindendes Fragment davon gemäß Anspruch 1, der die Proliferation und Differenzierung von Neutrophilen oder deren Vorläuferzellen anregt.

3. Agonist-Antikörper oder ein bindendes Fragment davon gemäß Anspruch 1 oder 2, der mit dem extrazellulären Teil des menschlichen G-CSF Rezeptors wechselwirkt.

4. Agonist-Antikörper oder bindendes Fragment davon gemäß einem der Ansprüche 1 bis 3, **dadurch gekennzeichnet, dass** das Immunogen, welches verwendet wird, um den Antikörper oder das bindende Fragment davon zu erzeugen, natives menschliches G-CSF oder eine Mutante davon mit Substitutionen, Insertionen oder Deletionen, umfasst, mit der Maßgabe, dass der resultierende Antikörper oder das bindende Fragment davon den menschlichen G-CSF Rezeptor bindet.

5. Agonist-Antikörper oder ein bindendes Fragment davon gemäß einem der Ansprüche 1 bis 4, der oder das an einem Epitop zwischen den Aminosäureresten 1 bis 603 (SEQ ID Nr.:27) des G-CSF Rezeptors wechselwirkt.

6. Agonist-Antikörper oder ein bindendes Fragment davon gemäß einem der Ansprüche 1 bis 5, der oder das ein monoklonaler Antikörper oder bindendes Fragment davon, wie z.B. F(ab)'₂, ist.

7. Agonist-Antikörper oder ein bindendes Fragment davon gemäß einem der Ansprüche 1 bis 6, wobei die CDRs der variablen Region der schweren Kette des Agonist-Antikörpers die folgenden Aminosäuresequenzen beinhalten: und wobei die CDRs der variablen Region der leichten Kette die folgenden Aminosäuresequenzen beinhalten: oder

8. Agonist-Antikörper oder ein bindendes Fragment davon gemäß einem der Ansprüche 1 bis 6, wobei die CDRs der variablen Region der schweren Kette des Agonist-Antikörpers die folgenden Aminosäuresequenzen beinhalten: und wobei die CDRs der variablen Region der leichten Kette des Agonist-Antikörpers die folgenden Aminosäuresequenzen beinhalten: oder

9. Agonist-Antikörper oder ein bindendes Fragment davon gemäß einem der Ansprüche 1 bis 6, der oder das an das gleiche Epitop wie der Agonist-Antikörper gemäß Anspruch 7 oder 8 bindet.

10. Agonist-Antikörper oder ein bindendes Fragment davon gemäß einem der Ansprüche 1 bis 9, wobei der Antikörper oder ein bindendes Fragment davon die Proliferation von Mensch- oder Mauszellen, exprimierend den menschlichen G-CSF Rezeptor wie bestimmt in einem *in vitro* Proliferationstest, anregt.

11. Hybridom-Zelllinie, die den monoklonalen Agonist-Antikörper oder das bindende Fragment davon gemäß einem der Ansprüche 6 bis 10 erzeugt.

12. DNA-Sequenz, codierend den monoklonalen Antikörper oder ein bindendes Fragment davon gemäß einem der Ansprüche 1 bis 10.

13. Gentransfersystem, beinhaltend die DNA-Sequenz gemäß Anspruch 12.

14. Gentransfersystem gemäß Anspruch 13, beinhaltend die DNA-Sequenzen, welche die Aminosäuresequenzen wie dargestellt in SEQ ID Nr.:15 bis 20 oder SEQ ID Nr.:21 bis 26 codieren oder welche ein bindendes Fragment davon gemäß einem der Ansprüche 1 bis 6 codieren.

15. Gentransfersystem gemäß Anspruch 13 oder 14, wobei das Transfersystem einen viralen Vektor, ein Plasmid oder ein nicht-vektorielles Transfersystem beinhaltet.

16. Verwendung des Agonist-Moleküls gemäß einem der Ansprüche 1 bis 10 zur Herstellung eines Arzneimittels zur Behandlung von Neutropenie.

17. Verfahren einer Hochdurchsatzdurchmusterung für einen Antikörper mit G-CSF Agonist-Aktivität, umfassend das Erstellen einer auf G-CSF ansprechenden Zelllinie, wie z.B. NFS60, mit einem Konstrukt, welches fähig ist zum Exprimieren eines Reportergens, wie z.B. Luciferase, β-Galactosidase, *Green Fluroescence Protein* oder Dihydrofolatreduktase, unter der Kontrolle eines auf G-CSF ansprechenden Promotors, und das Messen enzymatischer Aktivitäten oder Fluoreszenzdichten in den Zellen nach Anregung durch den zu testenden Antikörper.

18. Verfahren gemäß Anspruch 17, wobei der Test verwendet wird in Kombination mit einem colorimetrischen Testsystems, wie z.B. das Messen der Reduktion von 3-(4,5-Dimethylthiazol-2-yl)-2,5-Diphenyl-Tetrazoliumbromid (MTT), oder einem Aufnahmetest unter Verwendung von Zellen, exprimierend den G-CSF Rezeptor.

19. Verfahren gemäß Anspruch 17, wobei das Konstrukt die Sequenz der extrazellulären Domäne des G-CSF Rezeptors beinhaltet.

20. *In vitro* Verfahren zum immunologischen Nachweis eines menschlichen G-CSF Rezeptors mittels eines Antikörpers gemäß einem der Ansprüche 1 bis 10.

21. *In vitro* Verfahren zum immunologischen Nachweis einer Zelle, welche einen menschlichen G-CSF Rezeptor auf der Zelloberfläche exprimiert, mittels eines Antikörpers gemäß einem der Ansprüche 1 bis 10.

22. *In vitro* Verfahren zum immunologischen Nachweis und zur immunologischen Bestimmung eines löslichen menschlichen G-CSF Rezeptors mittels eines Antikörpers gemäß einem der Ansprüche 1 bis 10.

## Revendications

1. Anticorps agoniste ou fragment de liaison de celui-ci qui se lie spécifiquement à ou interagit avec le récepteur du G-CSF humain et le dimérise, dans lequel ladite dimérisation stimule la prolifération et la différentiation cellulaires.

2. Anticorps agoniste ou fragment de liaison de celui-ci selon la revendication 1 qui stimule la prolifération et la différentiation des neutrophiles ou de leurs cellules souches.

3. Anticorps agoniste ou fragment de liaison de celui-ci selon la revendication 1 ou 2 qui interagit avec la portion extracellulaire du récepteur du G-CSF humain.

4. Anticorps agoniste ou fragment de liaison de celui-ci selon l'une quelconque des revendications 1 à 3, **caractérisé en ce que** l'immunogène utilisé pour générer ledit anticorps ou fragment de liaison de celui-ci comprend le G-CSF humain natif ou un mutant de celui-ci avec des substitutions, des insertions ou des délétions, sous réserve que l'anticorps ou le fragment de liaison de celui-ci résultant se lie au récepteur du G-CSF humain.

5. Anticorps agoniste ou fragment de liaison de celui-ci selon l'une quelconque des revendications 1 à 4, qui interagit à un épitope entre les résidus d'acides aminés 1 à 603 (SEQ ID NO : 27) du récepteur du G-CSF.

6. Anticorps agoniste ou fragment de liaison de celui-ci selon l'une quelconque des revendications 1 à 5, qui est un anticorps ou fragment de liaison de celui-ci monoclonal tel que F(ab)'₂.

7. Anticorps agoniste ou fragment de liaison de celui-ci selon l'une quelconque des revendications 1 à 6, dans lequel les CDR de la région variable de la chaîne lourde de l'anticorps agoniste incluent les séquences d'acides aminés suivantes : et dans lequel les CDR de la région variable de la chaîne légère de l'anticorps agoniste incluent les séquences d'acides aminés suivantes : ou

8. Anticorps agoniste ou fragment de liaison de celui-ci selon l'une quelconque des revendications 1 à 6, dans lequel les CDR de la région variable de la chaîne lourde de l'anticorps agoniste incluent les séquences d'acides aminés suivantes : et dans lequel les CDR de la région variable de la chaîne légère de l'anticorps agoniste incluent les séquences d'acides aminés suivantes : ou

9. Anticorps agoniste ou fragment de liaison de celui-ci selon l'une quelconque des revendications 1 à 6, qui se lie au même épitope que l'anticorps agoniste de la revendication 7 ou de la revendication 8.

10. Anticorps agoniste ou fragment de liaison de celui-ci selon l'une quelconque des revendications 1 à 9, dans lequel l'anticorps ou un fragment de liaison de celui-ci stimule la prolifération de cellules humaines ou murines exprimant le récepteur du G-CSF humain ainsi qu'il est déterminé dans un test de prolifération *in vitro*.

11. Lignée cellulaire d'hybridome qui produit l'anticorps monoclonal agoniste ou un fragment de liaison de celui-ci tel que défini dans l'une quelconque des revendications 6 à 10.

12. Séquence d'ADN codant pour l'anticorps monoclonal ou un fragment de liaison de celui-ci tel que défini dans l'une quelconque des revendications 1 à 10.

13. Système d'administration de gènes incluant la séquence d'ADN selon la revendication 12.

14. Système d'administration de gènes selon la revendication 13 incluant les séquences d'ADN codant pour les séquences d'acides aminés montrées dans les SEQ ID NOs : 15 à 20 ou les SEQ ID NOs : 21 à 26, ou codant pour un fragment de liaison de celles-ci tel que défini dans l'une quelconque des revendications 1 à 6.

15. Système d'administration de gènes selon la revendication 13 ou 14, dans lequel le système d'administration inclut un vecteur viral, un plasmide, ou un système d'administration non vecteur.

16. Utilisation de la molécule agoniste selon l'une quelconque des revendications 1 à 10 pour la préparation d'une composition pharmaceutique pour traiter la neutropénie.

17. Procédé de criblage à haut rendement pour un anticorps ayant une activité agoniste du G-CSF comprenant la construction d'une lignée cellulaire réactive au G-CSF, telle que NFS60, avec une construction capable d'exprimer un gène rapporteur, telle que la luciférase, la β-galactosidase, la protéine de la fluorescence verte ou la dihydrofolate réductase, sous le contrôle d'un promoteur réactif au G-CSF et la mesure des activités enzymatiques ou des densités de fluorescence dans les cellules après stimulation par l'anticorps à tester.

18. Procédé selon la revendication 17, dans lequel le test est utilisé en combinaison avec un système de test colorimétrique tel que la mesure de la réduction du bromure de 3-(4,5-diméthylthiazol-2-yl)-2,5-diphényl-tétrazolium (MTT) ou un test de recaptage en utilisant des cellules exprimant le récepteur du G-CSF.

19. Procédé selon la revendication 17, dans lequel la construction inclut la séquence du domaine extracellulaire du récepteur du G-CSF.

20. Procédé *in vitro* pour détecter le récepteur du G-CSF humain immunologiquement au moyen d'un anticorps tel que défini dans l'une quelconque des revendications 1 à 10.

21. Procédé *in vitro* pour la détection immunologique d'une cellule exprimant le récepteur du G-CSF humain sur la surface de la cellule au moyen d'un anticorps tel que défini dans l'une quelconque des revendications 1 à 10.

22. Procédé *in vitro* pour détecter et déterminer un récepteur du G-CSF humain soluble immunologiquement au moyen d'un anticorps tel que défini dans l'une quelconque des revendications 1 à 10.


### SEQUENCE LISTING

<110> Baofu Ni
   Bill N.C. Sun
   Cecily R.Y. Sun
<120> G-CSF receptor agonist antibodies and
   screening method therefor
<130> 98-3
<150> 60/083,575
   <151> 1998-04-30
<160> 27
<170> FastSEQ for Windows Version 3.0
<210> 1
   <211> 22
   <212> DNA
   <213> Artificial sequence
<400> 1
   aagtggtgct atggcaaggc tg
   22
<210> 2
   <211> 23
   <212> DNA
   <213> Artificial sequence
<400> 2
   cactccagct gtgcccaggt ctt
   23
<210> 3
   <211> 37
   <212> DNA
   <213> Artificial sequence
<400> 3
   cccccccagc gctagcaata gcaacaagac ctggagg
   37
<210> 4
   <211> 29
   <212> DNA
   <213> Artificial sequence
<400> 4
   ggaattccta gaagctcccc agcgcctcc
   29
<210> 5
   <211> 17
   <212> DNA <213> Artificial Sequence
<220>
   <223> mouse
<400> 5
   aatacgactc actatag
   17
<210> 6
   <211> 38
   <212> DNA <213> Artificial sequence
<400> 6
   aggtcttgtt gctattgcta gcgctggggg ggcccagg
   38
<210> 7
   <211> 31
   <212> DNA
   <213> mouse
<400> 7
   atgggcwtca agatggagtc acakwyycwg g
   31
<210> 8
   <211> 20
   <212> DNA
   <213> mouse
<400> 8
   actggatggt gggaagatgg
   20
<210> 9
   <211> 30
   <212> DNA
   <213> mouse
<400> 9
   atggmttggg tgtggamctt gctattcctg
   30
<210> 10
   <211> 21
   <212> DNA
   <213> mouse
<400> 10
   cagtggatag acagatgggg g
   21
<210> 11
   <211> 30
   <212> DNA
   <213> mouse
<400> 11
   atggatttwc aggtgcagat twtcagcttc
   30
<210> 12
   <211> 20
   <212> DNA
   <213> mouse
<400> 12
   actggatggt gggaagatgg
   20
<210> 13
   <211> 25
   <212> DNA
   <213> mouse
<400> 13
   atgractttg ggytcagctt grttt
   25
<210> 14
   <211> 21
   <212> DNA
   <213> mouse
<400> 14
   cagtggatag accgatgggg c
   21
<210> 15
   <211> 5
   <212> PRT
   <213> mouse
<400> 15
   Asn Tyr Gly Met Asn
   1 5
<210> 16
   <211> 17
   <212> PRT
   <213> mouse
<400> 16
   Trp Ile Asn Thr Tyr Thr Gly Glu Pro Thr Tyr Ala Gly Asp Phe Lys
   1 5 10 15
   Gly
<210> 17
   <211> 12
   <212> PRT
   <213> mouse
<400> 17
   Glu Gly Phe Tyr Gly Gly His Pro Gly Phe Asp Tyr
   1 5 10
<210> 18
   <211> 17
   <212> PRT
   <213> mouse
<400> 18
   Lys Ser Ser Gln Ser Leu Leu Ser Ser Arg Thr Arg Lys Asn Tyr Leu
    1               5                   10                  15
   Ala
<210> 19
   <211> 7
   <212> PRT
   <213> mouse
<400> 19
   Trp Ala Ser Thr Arg Glu Ser
    1               5
<210> 20
   <211> 8
   <212> PRT
   <213> mouse
<400> 20
   Lys Gln Ser Tyr Asn Leu Arg Thr
    1               5
<210> 21
   <211> 5
   <212> PRT
   <213> mouse
<400> 21
   Ser Tyr Ala Met Ser
    1               5
<210> 22
   <211> 17
   <212> PRT
   <213> mouse
<400> 22
   Gly Ile Ser Ser Gly Gly Ser Tyr Ser Tyr Tyr Pro Gly Thr Leu Lys
    1               5                   10                  15
   Gly
<210> 23
   <211> 11
   <212> PRT
   <213> mouse
<400> 23
   Glu Ala Tyr Asn Asn Tyr Asp Ala Leu Asp Tyr
    1               5                   10
<210> 24
   <211> 10
   <212> PRT
   <213> mouse
<400> 24
   Arg Ala Ser Ser Ser Val Thr Tyr Val His
    1               5                   10
<210> 25
   <211> 7
   <212> PRT
   <213> mouse
<400> 25
   Ala Thr Ser Asn Leu Ala Ser
    1               5
<210> 26
   <211> 9
   <212> PRT
   <213> mouse
<400> 26
   Gln Gln Trp Thr Ser Asn Pro Phe Thr
    1               5
<220> 27
   <211> 603
   <212> PRT
   <213> human
<400> 27